# EUROPEAN PATENT SPECIFICATION

(11) **EP 1 917 576 B1**
(45) Date of publication and mention of the grant of the patent: **24.10.2012**
(21) Application number: 05785766.6
(22) Date of filing: 25.08.2005
(51) Int. Cl.: G06F 3/048, G06F 19/00

(54) **MEDICAL APPARATUS AND USER INTERFACE FOR A MEDICAL APPARATUS**
MEDIZINISCHE VORRICHTUNG UND BENUTZEROBERFLÄCHE FÜR EINE MEDIZINISCHE VORRICHTUNG
APPAREIL MEDICAL ET INTERFACE UTILISATEUR POUR APPAREIL MEDICAL

(43) Date of publication of application: 07.05.2008
(73) Proprietor: Gambro Lundia AB, 22010 Lund (SE)
(72) Inventor: MALAGOLI, Matteo, I-41012 Carpi (IT); MOLDUCCI, Fabrizio, I-41038 San Felice sul Panaro (IT); RONCADI, Fabio, I-41037 Mirandola (IT); ROYER, Michel, Ottawa, Ontario K1G0E7 (CA)
(74) Representative: Ponzellini, Gianmarco
(86) International application number: PCT/IB2005/002516
(87) International publication number: WO 2007/023329

(56) References cited:
- EP-A- 1 524 591
- US-A1- 2002 154 176
- US-A1- 2003 132 911
- INTERNATIONAL BUSINESS MACHINES CORPORATION: "Fully-navigable breadcrumb trails (or browsable breadcrumbs)" RESEARCH DISCLOSURE, MASON PUBLICATIONS, HAMPSHIRE, GB, vol. 461, no. 130, September 2002 (2002-09), XP007131244 ISSN: 0374-4353

## Description

### Technical Field

The invention relates to a medical apparatus and to a user interface for a medical apparatus. The medical apparatus of the invention could be an apparatus intended for the extracorporeal treatment of blood, for instance by hemodialysis, hemofiltration, hemodialfitration, ultrafiltration, plasmapheresis or an apparatus for processing whole blood and/or blood components.

### Background Art

Blood treatment apparatus and similar medical devices comprise an extracorporeal circuit, provided with at least one blood treatment or blood processing unit, one tube, connecting a blood removal zone to said unit, and a second tube, extending downstream of the treatment or processing unit towards a blood return zone to the patient or towards a blood/blood components collection zone. Blood is moved from the patient or donor to the treatment or processing unit via pumps or other actuators controlled by the machine.

In case the machine is for instance a hemodialysis apparatus, then for achieving the required treatment of blood, an adequately prepared dialysis liquid shall be sent to the treatment unit, and water removal through the treatment unit membrane as well other machine parameters shall be controlled. Depending upon the type of hemodialysis machine and upon the type of treatment, a user can have the possibility to set a number of parameters in order to impose a specific prescription to a patient, such as for instance: flow rates of the various liquids, temperatures and conductivity of the liquids, concentration of the liquids used during treatment, flow rates of any anticoagulants used and delivered during treatment, pressures in the fluid conduits, net liquid removal rates of plasma water from whole blood and so on.

It is therefore evident that users (the patient himself, a physician, a nurse) have normally a plurality of parameters to set before or even during the process performed by the medical apparatus.

In this situation easy and safe data entry as well as reliable data storage and transmission are particularly important in machines as blood treatment machines or blood processing apparatus where the process executed by the machine acts on a patient's or donor's blood. Particularly, in case of treatment of patients suffering from kidney failure, the patient is constantly connected to the machine with the serious risk that any failure in entering or in actuating a prescription could have negative impacts on the treatment delivery and on patient's health.

It is also to be noted that when setting up a blood treatment machine or similar apparatus, the user has to enter a relatively big number of treatment parameters and of machine operation parameters. This is accomplished giving the user possibility of navigation through various data entry screens wherein parameters of the same typology or parameters referring to a common context can be entered.

In this situation several technical solutions have been developed in the past in order to render parameters values data entry in blood treatment or blood processing apparatus relatively easy and reliable.

A data entry user interface of general purpose and not specifcally designed for the medical field is known from United States Patent 5,546,582.

A first known method for entering data in a dialysis machine is described in US 5247434. This method comprises the following steps:
(a) providing a touch screen interface with an indicium thereon corresponding to a treatment parameter;
(b) touching the indicium;
(c) in response to said touching, invoking a data entry pad on a region of the touch screen;
(d) entering a parametric value corresponding to the treatment parameter by touching one or more buttons of the data entry pad;
(e) touching a first region of the data entry pad to signal entry of the parametric value;
(f) displaying on the touch screen a button soliciting verification of the newly entered parametric value; ,
(g) touching the button soliciting verification; and
(h) In response to steps (b)-(g), causing the parametric value corresponding to the treatment parameter to be changed.

In other words before really implementing a change a user is solicited to verify the newly entered parameter and to press a button confirming the change.

The same patent also discloses a method for entering variable parameters, i.e. parameters that can vary in the course of time during treatment.

More in detail US5247434 shows a method of programming a time-varying parameter comprising the steps:
(a) providing a touch screen interface;
(b) displaying on the touch screen first and second axes, the first axis corresponding to the time-varying parameter, the second axis corresponding to time;
(c) touching the touch screen at a plurality of points to define points on a parameter-versus-time curve;
(d) presenting on the touch screen a series of bars corresponding to said curve;
(e) selecting one of said bars for alteration;
(f) displaying on the screen a numeric parameter corresponding to the selected bar;
(g) touching the screen at first or second locations to increase or decrease, respectively, the displayed numeric parameter and thereby alter the value of the numeric parameter to which the selected bar corresponds;
(h) touching the screen at a third location to signify completion of steps (b)-(g); and
(i) storing data corresponding to the bars in a memory to which the process-control system can refer in changing the time-varying parameter with time.

Document US5326476 teaches a further method for entering a time variable parameter, ultrafiltration in particular, in a hemodialysis machine, having a programmable memory and having ultrafiltration capability, so as to enable the machine to perform ultrafiltration of fluid from a patient according to a time-variable ultrafiltration profile. The method disclosed in US5326476 comprises the following steps:
(a) entering into the programmable memory a prescribed time for dialysis;
(b) entering into the programmable memory a target ultrafiltration volume of fluid to be removed from the patient;
(c) entering into the programmable memory a proposed ultrafiltration profile being representable as a plot of coordinates, on an ultrafiltration rate axis and a time axis and defining a profile ultrafiltration volume; and
(d) shifting the proposed ultrafiltration profile along the uftrafiltration rate axis to the degree necessary to make the profile ultrafiltration volume equal to the target ultrafiltration volume, so as to allow the hemodialysis machine to achieve, while ultrafiltrating the fluid according to the shifted ultrafiltration profile, the entered target ultrafiltration volume within the entered prescribed time.

This method allows the user to enter a profile curve and to move the ultrafiltration profile along the ordinates so as to achieve the desired integral value In the desired time frame.

A further user interface system for a dialysis machine is known from document US5788851 and comprises:
- a touch screen displaying messages and information and permitting to select a parametric value pertinent to operation of said machine or pertinent to a treatment by said machine,
- one hard key off of said touch screen, said touch screen prompting a user to press said hard key to signify that the selection of the parametric value has been completed;
- a control system having a host and a safety processing unit, wherein pressing of said hard key causes transfer of information relating to the selected parametric value from the host processing unit to the safety processing unit which is then checking said selected parametric value to confirm that said parametric value meets validation or safety criteria for a patient connected to said machine.

The above system is therefore using two processing units to avoid that unsafe parameters values are entered into the machine.

It is also known from US6811707 using a dialysis machine wherein after entry of a value for a parameter the value is stored in at least two different memory locations connected to corresponding separate main control units. At predetermined intervals of time, one of the units sends the dialysis treatment parameters values stored In its memory to the other unit, The values are then compared and an alarm signal generated if the values of the same parameters in the two memories of the two units do not coincide with each other.

Finally it is known to adopt on dialysis machines a visualization of both the value set by the user for a parameter and the value actually achieved by the machine for the same parameter. This double visualization is however done in a way such that the user has to navigate through several menus or displays of the user interface to have access to both the set information and the actually measured parameter value.

EP1524591 shows a navigation structure with a plurality of tab elements and with capability of detecting selection of one of the tab clements in the navigation structure. If the selected tab has one associated element the navigation structure is changed by deleting the non-selected tab elements and adding to the structure tab elements associated with the selected clement.

### Summary of the invention

While the above disclosed systems and methods served to give the user possibility to enter data in a relatively easy manner and/or served to reduce data entry errors or data memorization errors, the present invention aims to further improve ease and reliability in data entry procedures for medical devices and particularly for blood treatment or blood processing machines where easy and intuitive navigation is essential to speed up and render reliable setting of machine.

In this context, the invention aims to offer a reliable and easy system for navigation through the plurality of screen a user shall access in order to complete a medical apparatus set up procedure and/or in order to visualize all the information typically required when working on complex medical devices.

In particular, the invention aims to provide the user with an improved navigation system particularly for touch screen based user interfaces of medical apparatus wherein the user is provided with clear information concerning the hierarchical structure of the interface and of the various screens and environments that the user interface allows to access.

In accordance with a further aspect of the invention, it is an aim of the invention providing users with easy to access and read information relating to both set values and measured values for parameters.

The above aims are reached by a medical apparatus according to the appended claims, Further characteristics and advantages will better emerge from the following description in relation to some preferred but non-exclusive embodiments of an apparatus according to the invention.

### Short description of the drawings

The description will be made with reference to the figures of the accompanying drawings, provided by way of non-limiting example, in which:

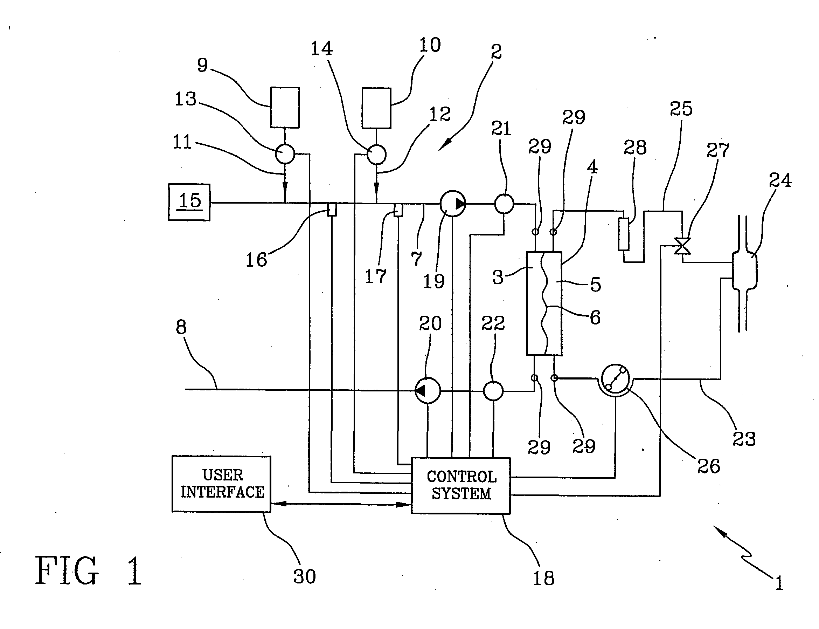

- figure 1 is a schematic representation of a medical apparatus, for instance a blood treatment machine ;
- figure 2 schematically shows a user interface for use on a medical apparatus according to the invention;
- figures 3 to 6 schematically show possible displays which can be visualized on a screen portion of the user interface according to the invention,
- figures 7 and 8 show the user interface of figure 2 in other operating conditions, namely while the apparatus of figure 1 is in priming mode and in dialysis treatment mode.

### Detailed Description

With reference to the figures, reference number 1 denotes a medical apparatus.

The medical apparatus of the shown embodiment is a machine for the extracorporeal treatment of blood. Of course the medical apparatus of the invention could alternatively be a blood processing device or a blood component preparation device or other medical apparatus for fluid delivery/collection.

The apparatus shown in the enclosed drawings comprises a module 2 for preparing dialysis liquid to be sent Into a first chamber 3 of a blood treatment unit 4, which is formed by a casing defining at least two chambers 3, 5 separated by a semipermeable membrane 6. The dialysis preparation module 2 includes tubing 7 bringing dialysis liquid to the first chamber inlet, while a waste line 8 receives spent liquid exiting via an outlet of the first chamber. In detail, the module 2 includes one or more concentrate containers 9, 10 delivering concentrated solutions, via respective lines 11, 12 and upon the action of respective concentrate pumps 13, 14, into the tubing 7 thereby properly mixing water coming from a source 15 with said concentrates and obtaining the dialysis liquid. Conductivity or concentration sensors 16, 17 can be provided on tubing 7 downstream each respective concentrate line. Said sensors provide control signals to a control system 18 which is responsible to then act on the concentrate pumps. A pump 19 is generally operating on tubing 7 and a pump 20 on the waste line 8. Of course different alternative embodiments can be envisaged to bring dialysis liquid to the treatment unit with appropriate chemical and physical properties. For instance pre-prepared dialysis liquid bags could be used with no need of online preparation of dialysis liquid starting from concentrates and water. Fluid balance sensors, for instance a first and a second flow-meter 21, 22, operating on tubing 7 and on waste line 8 respectively, are used and are connected to the control system to provide signals necessary for regulating at least one of pumps 19, 20. Of course other fluid balance systems can be used: scales for instance or balance chambers.

When the apparatus is in use, an extracorporeal blood circuit is connected to the second chamber 5. The extracorporeal circuit usually comprises at least an access branch 23 extending between a blood removal zone 24 from a patient or donor and the treatment unit 4, at least a return branch 25 extending downstream of the treatment unit, between the second chamber and a return zone of the blood to the patient; a peristaltic pump 26 is operatively associated to a length of pump tube of the extracorporeal circuit access branch. A clamp or other closure device 27 can operate on the blood return branch 25, typically downstream from a gas separator 28.

Usually, at the removal and return branches of the blood to or from the patient, access means are provided to the cardiovascular system of the patient, for example constituted by needles of appropriate sizes, catheters or accesses of different types. One or more liquid infusion lines could be provided connected at one end to an infusion liquid source (a bag or in-line infusion liquid preparation system) and at the other end to the extracorporeal circuit, or directly to the patient or donor.

Other sensors, such as pressure sensors 29, can be provided either on the extracorporeal circuit and/or on the dialysis liquid side of the apparatus.

The medical apparatus 1 can of course include other components, which are not herein disclosed as they are not relevant for the purpose of the present invention.

### User Interface

The apparatus 1 presents at least a user interface 30 for enabling setting of a plurality of parameters pertinent to operation of said apparatus or pertinent to a treatment to be performed by said apparatus. While the user interface herein disclosed is connected with and part of the apparatus 1, it shall be understood that the user interface 30 could be manufactured and sold separately and then connected to a medical apparatus.

The user interface 30 includes a touch-screen 31, which allows interaction with the user interface, for instance selection of certain parameters, visualization of values of said parameters and display of other information as it will be here below in detail described.

The activity of the user interface is determined by control system 18, which is connected to the user interface, is responsive to actions by a user on said user interface, and controls operations of the medical apparatus 1 by acting on a plurality of actuators (such as pumps 13, 14, 19, 20, 26, clamp 27 and others) and by receiving signals by a plurality of sensors (such as for instance sensors 16, 17, 21, 22, 29 etc.).

The control system includes a main control unit, connected to the user interface 30, and at least a memory connected to the main control unit. From a technical point of view the main control unit includes at least microprocessor, while the above-mentioned memory can be in a single physical memory or in physically separated memory devices.

The control system 18 is programmed (for instance by loading a program in the memory which then is executed by the CPU) for carrying out the following steps.

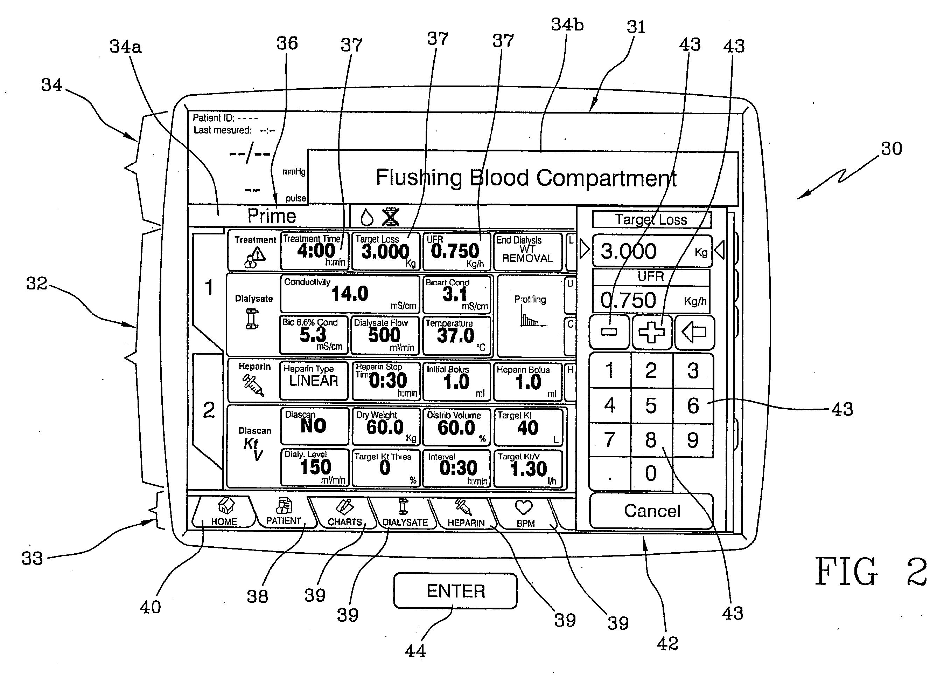

First the control system defines on screen 31 an operating region 32, where a number of working displays can be displayed, and a navigation region 33, where a number of navigation keys can be displayed (see figure 2). The working displays are shown one per time according to a sequence, which depends upon the sequence of activation of the navigation keys, as it will be apparent from below description.

The operating region 32 of the embodiment in figure 2 is adjacent to and in contact with the navigation region 33 and, in particular, is placed between the navigation region and a status bar region 34 extending transversally in the top part of the screen 31.

The navigation region of the non-limitative embodiments shown is in the bottom part of the screen and contains a plurality of navigation keys 35.

Of course the relative positions of regions 32, 33 and 34 could be differently arranged without departing from the scope of the invention. The control system displays In the operating region a plurality of working displays 36, each including one or more indicia, such as touch buttons or touch icons 37, which can be selected to access particular submenus and/or for selecting a parameter or a group of parameters for successive modification of the value(s) thereof.

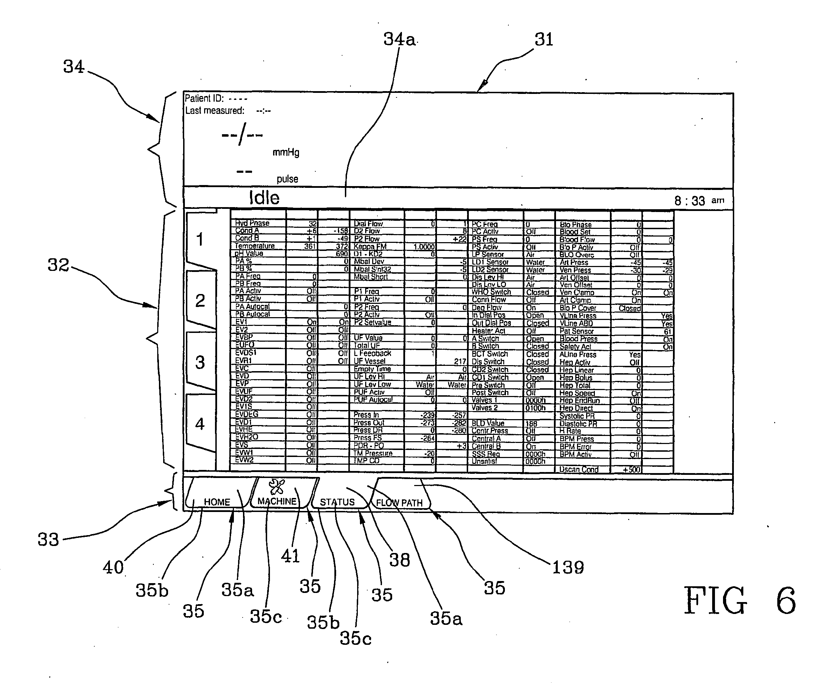

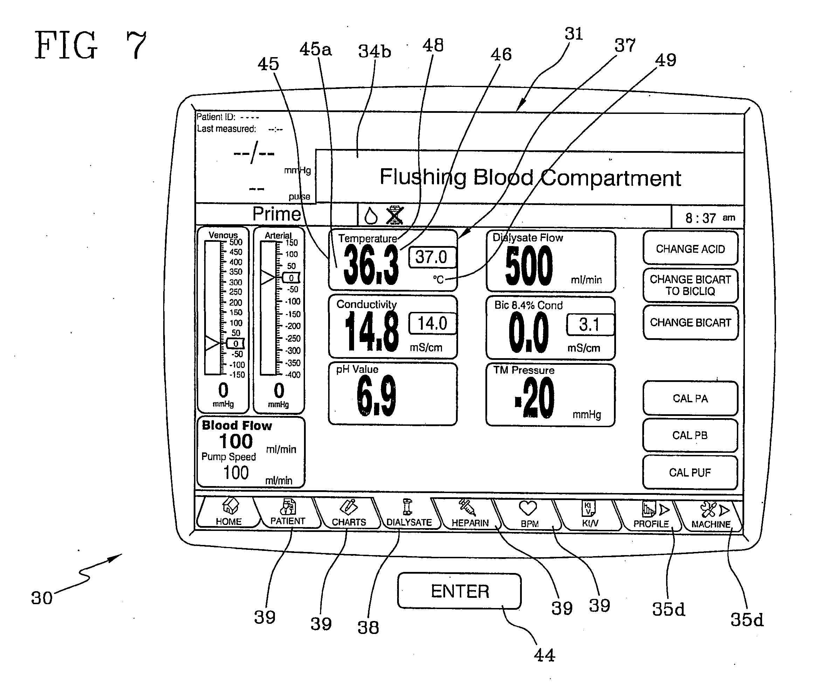

In practice, the medical apparatus can be in different operating modes, for instance by way of non-limiting example idle mode, setup mode, priming mode, treatment (or dialysis) mode, and rinse-back mode. The status region 34 identifies in a band 34a information relating mode, while the operating region is accessible to the user for entering commands or for modifying settings of one or more parameters (see figures 2 and 6). Each operating region contains working displays displaying information, in alphanumeric or in analogical or in graphic form, regarding the status of the machine, the current actual values of one or more parameters, etc. When displayed according to the technique here below described, each working display occupies the entire operating region, overlapping to or hiding previously displayed working displays. The status bar in addition to information concerning the status mode of the machine (flushing, treatment, priming, rinse back, etc.) or can also provide alarm or information messages 34b (see figures 2, 7).

The control system is programmed to associate to each of said number of navigation keys 35 a corresponding working display 36 so that each working display is selectable and displayable in the operating region upon selection of the corresponding navigation key. In other words the control system links the navigation key to one corresponding working display and therefore loads from a memory and displays on the operating region of the screen the working display associated to the navigation key selected by the user. Moreover the control system organizes a plurality of navigation keys and corresponding working displays according to a multi-level hierarchical organization where one root is present an where, upon selection of the navigation keys, it is possible for the user to access the several branches and sub-branches deriving from the root.

In this hierarchical organization the following relations are defined:
1. Selected key 38 is the key selected by the user;
2. Ancestor-keys 40, 41 of a selected key 38 (other than the ROOT key) are defined as keys hierarchically one or more levels above the selected key;
3. Siblings 39, i.e. brothers, of a selected navigation key 38 (other than the ROOT key) are defined as keys hierarchically at the same level of said selected navigation key;
4. Son-keys 139 of a selected navigation key 38 are defined as keys hierarchically one level below the selected key.

The control system is, in use, programmed to display in the navigation region at least the following keys:
- the key 38 selected by default or the user, '
- if present, one or more son-keys 139 of the selected key, and
- if present, one or more siblings 39 of the selected key,
- if present, one or more ancestor-keys 40,41.

The control system of the embodiment shown is in detail programmed to manage navigation based on the following rules (other embodiments could adopt only part of the following rules):
1. The highest in level key, or root key, is always present on the screen and displayed in the region 33.
2. At startup, one default key is selected; in the embodiment herein described the root key is the one the control system selects by default at the beginning of any session.
3. During navigation a single path in the hierarchy is identified: in other words in order to select a certain key within the hierarchy, it is necessary to first select the proper and unique sequence of ancestor keys of said key.
4. All keys belonging to the same level in hierarchy of a selected key (i.e. siblings) are simultaneously displayed on the screen, along with all ancestor keys of the selected key, unless the selected key has son-keys.
5. In case the selected key has son-keys, then all of the keys belonging to the same level of the selected key (if they exist) are hidden; the selected key is displayed together with the key representing the next higher level in the hierarchy (if present) and with all keys belonging to the next level lower in the hierarchy (i.e. the son keys).
6. The working display corresponding to the selected key is displayed in the operating region 32 of the screen during navigation.

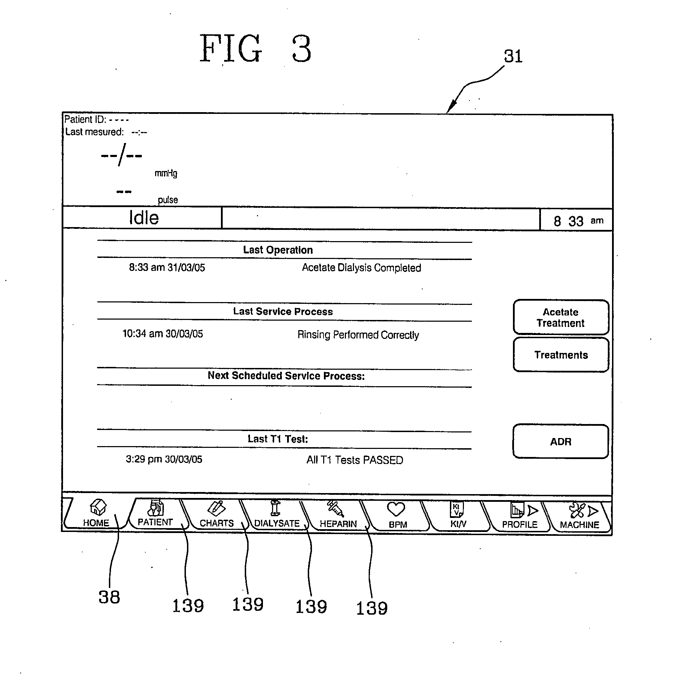

A possible way of working of a control system in accordance with the invention in response to actions of the user on a key is described in below table referring to figures 3 to 6 which all relate to a situation wherein the apparatus is in idle mode.

| **Steps** | | |
|---|---|---|
| **Operator Action** | **Control system Response** | |
| 1. The operator selects a key by pressing it (for instance one of keys 39 in figure 3 - notice that in figure 6 the home key is by default the selected key 38) | | |
| | 2. IF the newly selected key contains at least one son-key, the control is programmed to: | |
| | | a. Hide all of the keys belonging to the same level of the newly selected key (if they exist) |
| | | b. Display the selected key on the immediate right of the key 40 representing the next level in the hierarchy (if present) higher than the level of the selected key |
| | | c. Displays all of the keys belonging to the next level in the hierarchy lower than the level of the selected key to the right of the selected key (if they exist). |
| | | d. Displays the working display corresponding to the selected key in the operating region 32 of the screen during navigation the corresponding screen |
| | ELSE (if there are no son keys as in figure 4 example): | |
| | | a. Displays the working display corresponding to the selected key in the operating region 32 of the screen |
| | | b. Displays all of the keys 39 belonging to the same level of the selected tab (if they exist) |

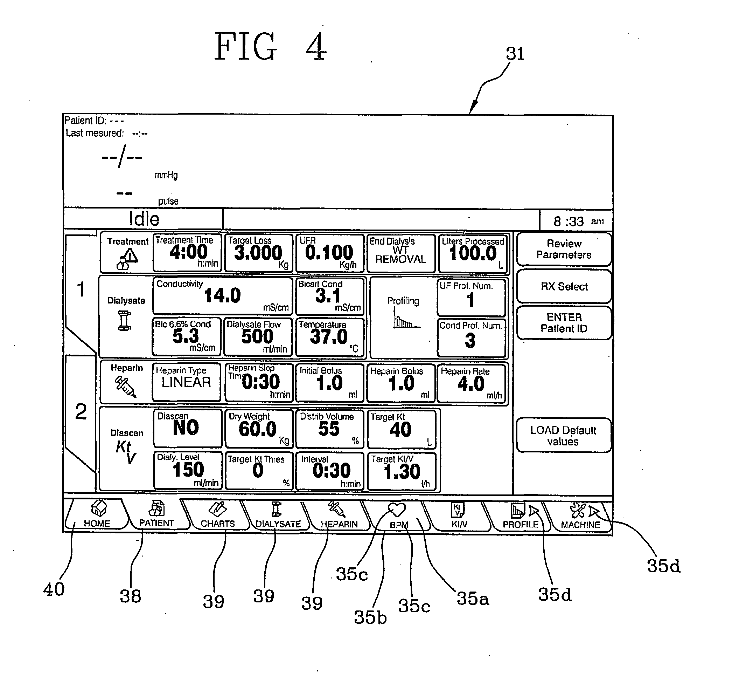

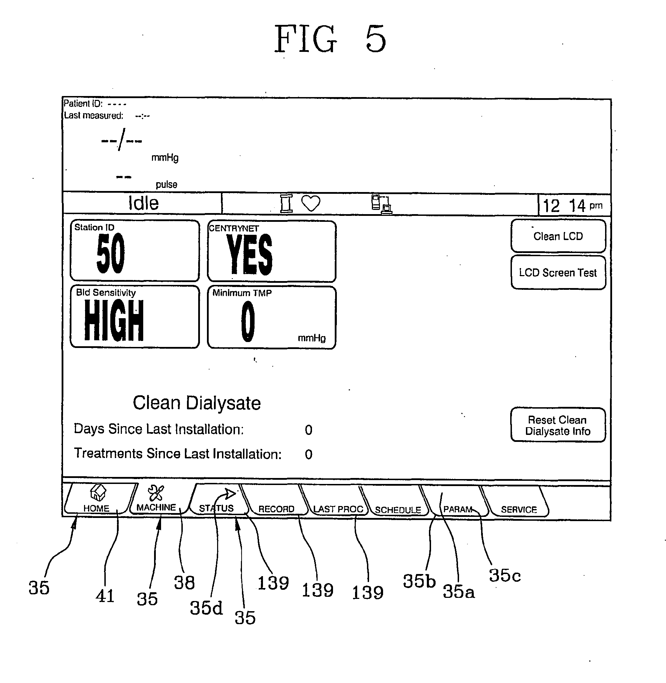

In order to move from the screen of figure 3 or of figure 4 to the screen shown in figure 6, the user has to first select the key labelled 'MACHINE' (this key is a son key 139 of the selected root key 38 if the user is in the situation shown in figure 3 or the same MACHINE key is a sibling key 39 of the selected key 38 if the user is in the situation shown in figure 4); then the user has reached the situation of figure 5 and has to press the key labelled 'STATUS' (in figure 5 this key is a son key 139 of selected MACHINE key 38, while in figure 6 the same 'STATUS' key is the selected key 38 and has one son key 139). Notice that in figure 6 the screen 31 of the user interface includes a navigation bar 33 displaying four navigation keys 35 where keys 40 and 41 are ancestors of the selected key 38 and where key 139 is son of the same selected key 38. Since the selected key 38 (STATUS key) in figure 6 has at least one son key 139, then any possible siblings of the selected key are hidden. Comparing the situation of figure 6 to the one of figure 2 we see that in figure 2 the navigation region shows a selected key 38, only one ancestor key 40 of said selected key and a number of sibling keys 39 of the selected keys since no son-keys 139 are present. The ancestor key 40 in figure 2 is also a root key in the multilevel hierarchical organization having no respective ancestor keys one or more levels above.

As shown in the figures, the keys 38 selected by the user are visually differentiated compared to the other keys in the navigation region and selection of said keys determines, in the operating region, displaying of at least the working display 36 associated to the selected key.

As already mentioned, the control system comprises a microprocessor based control unit, which is programmed to display the selected key in a manner visually differentiated compared to the sibling keys, the son-keys and to the ancestors-keys. Moreover both the sibling and the son-keys are displayed in a manner visually differentiated compared to the ancestor-keys.

Furthermore, the control unit is programmed to keep track of the group of ancestors-keys that need to be selected to allow access to and display of the selected key; the control system displays said group of ancestor-keys during the key display step.

Referring again to figure 6, the user starting from the root key 40, had then to select the key 41 to have possibility of selecting the key 38. Both key 40 and key 41, which are ancestor keys of selected key 38, are tracked by the control system and are displayed in a position adjacent to the selected key. According to the embodiment of figure 6, the closer the level of the ancestor key to the level of the selected key, the closer said ancestor key being displayed to the selected key: practically the 'father' key 41 is just adjacent to the selected key and the 'grandfather' key 40 is displayed adjacent to the 'father' key 41 of the selected key 38.

To further enhance an immediate and intuitive perception by the user during the keys display step, the control unit is programmed to display the group of ancestor-keys and the group of son-keys (or of sibling keys if the selected key has no son-keys) in opposite and adjacent positions relative to the selected key, as again visible in figure 6.

Again referring to the embodiment of the enclosed figures, it is shown that the control system is programmed to associate to each of said keys a number of variable properties including: a key background 35a, a key shape 35b, and a key identifier 35c (this latter is not necessarily always present).

In detail, during the keys display step the control system is programmed to display the key background 35a in a first condition, to indicate a selected key, to display the key background 35a in at least a second condition visually differentiated from the first condition, to indicate son-keys or sibling keys. The control system can also display the key background 35a in a third condition visually differentiated from the first and second conditions, to indicate ancestor-keys. The key background conditions can be differentiated by the colour/tone of the key or of a portion of the key, or by appropriate texturing and or rendering of the key or of a portion of the key. In the enclosed embodiment three different colours or tones (depending as to whether the screen allows use of colours or not) have been used: one for identifying the selected key, one for the son-keys and one for any ancestor keys. Sibling keys background is the same as son-keys background.

Notice that in principle a fourth background (colour/tone or texturing or rendering) could be used to differentiate son keys and sibling keys. This has not been adopted in the enclosed embodiment since if a selected key has son-keys, then any possible sibling key of the selected key is not displayed.

Moreover, during the keys display step, the control unit is programmed to vary also the key shape 35b depending upon the circumstances: ancestor-keys are displayed in a first shape and son-keys in a second shape visually differentiated from the first shape. Furthermore the control unit is programmed to configure the selected key according to said first shape, if the selected key has selectable son-keys (see figure 3 for instance), or according to said second shape, if the selected key has no selectable son-keys (see figure 2). In practice, if a key 39 or 139 has no son-keys, its shape (i.e. its inclination in the enclosed figures embodiments) does not change when it is selected.

As shown In the embodiments of the attached figures, each key is tab-shaped: in such a case, the first shape is a first parallelogram and the second shape a second parallelogram. This second parallelogram has two parallel sides, both parallel to two corresponding parallel sides of the first parallelogram, and two sides oriented transversally relative to corresponding two parallel sides of the first parallelogram. Basically the parallelogram shaped tabs can have two shapes, which are different for the inclination of two of their four sides.

Once again, the keys shape namely the inclination of the tabs in the shown embodiments is used to help identify the tab hierarchy. If the selected tab has son-keys, it and all tabs representing higher levels in the hierarchy are inclined according to the same angle. Levels lower than the selected tab have inclination that is the mirror image of the selected tab. If a tab has no children, its inclination does not change when it is selected.

Of course keys shaped according to two different shapes could be obtained by modifying the length of one or both the dimensions of tabs, or in other equivalent manner if non-tab shaped keys are being used. For instance tabs or keys having asymmetric shape could serve for the purpose of differentiating two series of keys/tabs.

Finally the control unit is programmed to display a key identifier 35c, which can be one in the group comprising an icon, a wording, or an alphanumeric representation, and wherein during the keys-display step the key identifier is displayed on the key surface for describing the function of or the information contained in the working display associated to each respective key.

A further feature of the present invention provides for the control system being programmed for identifying if one or more of the keys, which are displayed together with the selected key, i.e. son-keys if any or sibling keys, present on their turn respective son-keys. Notice that son-keys of son-keys (or of sibling keys) of a selected key are hidden when displaying the selected key In the navigation region. However, the control system is programmed to display a sublevel-indicium 35d on each son-key (or sibling key) having respective son-keys; in this way a user can have a perception not only of the number of son-keys or sibling keys selectable after having selected a certain key but also an information as to whether the son keys (or the sibling keys) have respective son-keys one level below (see figure 4 where several sibling keys 39 of the selected key 38 are shown: two of these sibling keys 39 present the indicium 35d showing presence of respective son keys).

In practice, referring for instance to figure 3, the control system is programmed for displaying the selected key and the corresponding working display on screen. Each time a different key is selected a corresponding working display is retrieved from a memory, part of the control system, and displayed in the operating region in place of the working display of the key previously selected. From a practical point of view each time a working display is displayed in the operating region it either hides the previous working display or the previous working display is first blanked and the new display displayed.

The control system detects selection of one son-key and transforms the selected son-key into a newly selected key, while transforming the previously selected key into an ancestor-key (see figures 5 and 6). Correspondingly, also the son-keys (if present) of the newly selected key are displayed in the navigation region and become available for selection. In case one of these new son-keys is selected the process just described is repeated. If no son keys are present then the control system displays any sibling key (see figures 3 and 4).

According to a possible embodiment the screen is a touch screen including said operating and said navigation regions; in this case the control unit allows selection of a key by detecting a touching of the corresponding area delimited by the key in the navigation region. Other techniques for key selection could be possible, for instance adopting a mouse or a stick cooperating with a pointer on screen.

As mentioned, when one key is selected at least one of said working displays is invoked on screen showing one or more indicia for identifying a parameter a user intends to modify. In figure 2 for instance, selection of the patient tab-key 38 causes invocation of the working display 32, which includes a plurality of indicia 37 such as the one for selection of treatment time, the one for selecting target weight loss, the one relating to UFR setting etcetera.

The control system is programmed for detecting touching of indicium 37. Touching of the indicium signifies selection of the indicium, identifies the parameter a user intends to modify (for instance in figure 2 the indicium 37 for modifying the target weight loss is selected and this means the user is then allowed to modify the target weight loss value), and allows setting of a new value for said parameter. Practically, in response to the touching of the indicium, the control system invokes a data-entry display 42 on the touch screen having at least two set buttons 43 such as buttons for each digit and-or buttons for increasing or decreasing the current value of the selected parameter. The display 42 could include an enter key 44 for entering the selected value. In other words the enter key can be either a touch sensitive area of the data entry display 42 or of the rest of the touch screen or a hard key off the touch screen. Once the new value has been set and entered, said new value is stored in a memory.

Alternatively to what above described the setting of a new value can be done as follows. The parameter is selected by a touching of indicium 37 on the active working display, and then in response to said touching, at least two set buttons and an enter key are activated on the touch screen working display (these keys could also be always available and active if enough room is available on the working display); then the user interface is controlled so that setting a of a new parametric value corresponding to the treatment parameter is obtained by touching one or more times the mentioned buttons (which can be shaped as arrow keys or + and - keys) and by detecting the touching of said enter key for signifying setting has been completed.

Referring to the embodiments shown, the interface 30 is associated to the extracorporeal blood treatment apparatus 1 and the parameters that can be selected and modified are one or more selected in the group comprising:
Temperature of the dialysis liquid,
Conductivity of the dialysis liquid.
Electrolytes concentration of the dialysis liquid,
Flow rate of the dialysis liquid,
,Flow rate of the spent dialysate,
Flow rate of the blood in the in one of said tubing,
Ultrafiltration rate through the semipermeable membrane,
Net weight loss rate,
Treatment time,
Weight loss,
Anticoagulant delivery rate and mode,
Infusion rate of substitution solution,
Infusion volume of substitution solution.

Given the number of parameters involved in the machine setup and configuration, the user interface and the navigation concept of the invention result in a great enhancement in term of easy, quick and reliable data entry.

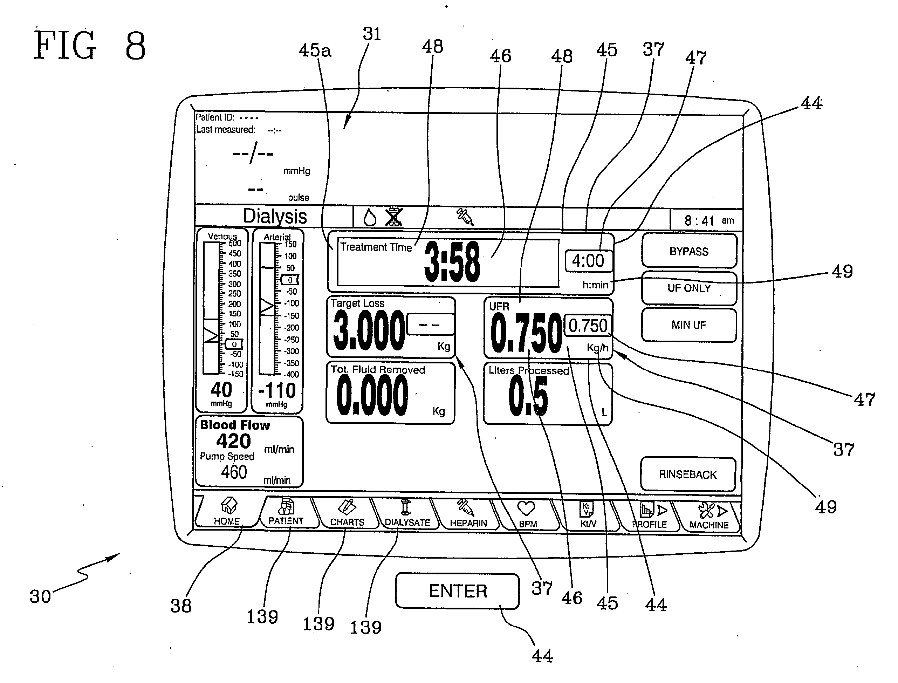

According to another aspect of the invention, which could be combined to the above features but also constitute a separate and independent concept, the plurality of parameters which can be set by the user through the user interface include a sub-group of parameters which are displayed as follows. This subgroup of parameters include one or more of the following parameters: treatment time, total weight loss (target loss in figure 8) to be achieved during said treatment time, temperature of the dialysis liquid, conductivity of the fresh dialysis liquid (refer also to figure 7). These parameters are set by the user but the machine needs time to react to the user setting and, therefore, it is possible to have discrepancies or differences between the value set by the user and the value actually present or achieved by the machine. This is very clear for conductivity or temperature of the dialysis liquid (figure 7). According to one aspect of the invention the current actual value and the set value of each one of the above parameters is displayed contemporaneously in the touch sensitive area of the indicium 37 that the user shall touch to modify the set value of the same parameter. More in detail, in case the user interface includes a touch screen, touch sensitive indicia 37 are defined on the touch screen for each parameter the user is allowed to select and modify in term of value (liquid temperatures, flows etc.). Each touch sensitive indicium, such as a button, comprises a border 45 delimiting a touch sensitive area 45a, which when the indicium is touched moves from a first to a second condition visually differentiated from the first condition. For instance in the second condition the indicium appears, depressed and/or differently coloured compared to the first condition.

Moreover, in case the indicium is related to one of parameters of said sub-group, the touch sensitive area of the button includes one or more of the following display regions:
- a first region 47 displaying the value a user has set for the parameter,
- a second region 46 displaying the current actual value for the same parameter as measured by one or more sensors connected to the control unit of the machine 1
- a third region 48 comprising an alphanumeric description of the parameter corresponding to the indicium, and
- a fourth region 49 displaying a unit of measure of the value of the parameter.

In this way the user selects the parameter by touching the button, modifies the set value for the parameter, and has on the surface of the same button both visualization of the setting and of the current real value for the same parameter. This enhances usability as the operator has both displays in the region of his/her maximum attention, i.e. the touch sensitive area, which requires the touch to start the parameter value modification sequence. Moreover since the two values are contemporaneously and closely displayed the user has immediate feedback as to whether the machine reacts or not to the new setting with no need of any other navigation through the interface screens.

### Software program product

It is also described a software program comprising instructions which, when executed by the main control system of the apparatus 1 or of the user interface, programs the control system to execute the steps which have been already disclosed in the chapter *'user interface'* and therefore not herein repeated.

More in particular the software program is able to program the control system associated to a user interface either of the above medical apparatus 1 or associated to a treatment apparatus of other nature so that the control system of the user interface or of the apparatus is programmed for executing the following main steps:
- defining on the user interface screen an operating region, where a number of working displays can be displayed, and a navigation region, where a number of navigation keys can be displayed,
- organizing the navigation keys according to a multi-level hierarchical structure where son-keys of a specific navigation key are defined as keys hierarchically one level below said specific key, and ancestor-keys of a specific key are defined as keys hierarchically one or more levels above the specific key;
- associating to each of said number of navigation keys a corresponding working display;
- verifying selection of a navigation key;
- displaying in said navigation region at least the following keys:the key selected by the user, if present, one or more son-keys of the selected key, and if present one or more ancestor-keys;
- displaying, in the operating region, at least the working display associated to the selected key.

Of course the software program can program the apparatus or the user interface control system to also execute each one of the other steps already described in the chapter *'user interface'* and not here below repeated.

The software program can be stored in any adequate support and then sold separately from the medical apparatus 1. In practice the software program could be stored on a magnetic recording support (for instance an hard disk, a cassette, a floppy disk, etcetera), or on an optical recording support (DVD or CD or other), on an electrical or electromagnetic carrier signal (if for instance the program is sent via a network), or on a computer readable memory (ROM, EPROM, RAM), or other convenient support memory device and then associated to the machine control system which running the program stored on said support is then programmed to render available a user interface having the features above described.

### Method for enabling setting up of a medical apparatus

Finally it is also described a method for setting up a medical apparatus of the type including at least a user interface with a screen (for instance a touch screen) for enabling setting of a plurality of parameters pertinent to operation of said apparatus or pertinent to a process to be performed by said apparatus.

The method comprises the following steps:
- defining on said screen an operating region 32, where a number of working displays 36 can be displayed, and a navigation region 33, where a number of navigation keys 35 can be displayed;
- associating to each of said number of navigation keys a corresponding working display, which is selectable upon selection of the corresponding navigation key;
- creating a multi-level hierarchical organization where:
   o son-keys 139 of a specific selected navigation key 38 are defined as keys hierarchically one level below said specific key and accessible for selection only upon selection of the specific key, and
   o ancestor keys 40, 41 of a specific key are defined as keys hierarchically one or more levels above the specific key 38 and which need to be selected for allowing selection of the specific key;
- displaying in said navigation region at least the following keys:
   o the key 38 selected by the user,
   o if present, one or more son-keys 39 of the selected key, and
   o if present one or more ancestor-keys 40, 41;
- displaying, in the operating region, at least the working display associated to the selected key, said working display comprising one or more indicia 37 for identifying one or more corresponding parameters a user may want to modify;
- allowing selection of at least one indicium for identifying the parameter a user intends to modify,
- allowing setting of a new value for said parameter;
- storing the new value in a memory.

To render the navigation intuitive and to give an immediate and clear perception of the navigation in the multilevel environment, during the keys display step the selected key is displayed in a manner visually differentiated compared to the son-keys and to the ancestors-keys and the son-keys are displayed in a manner visually differentiated compared to the ancestors-keys.

According to an aspect of the invention, the method provides for a step of keeping track of the group of ancestors-keys to be previously selected to then allow display of the selected key. This group of ancestor-keys is displayed during the key display step in a position adjacent to the selected key. In detail, the closer the level of the ancestor key to the level of the selected key, the closer said ancestor key is displayed to the selected key. To further enhance usability ancestor-keys 40,41 and son-keys 139 are displayed in opposite and adjacent positions relative to the selected key.

According to a further aspect the method provides for associating to each of said keys a number of variable properties including: a key background 35a, a key shape 35b, and a key identifier 35c.

In detail, during the keys display step the method provides for displaying the key background 35a in a first condition, to indicate a selected key, for displaying the key background 35a in at least a second condition visually differentiated from the first condition, to indicate son-keys, and for displaying the key background 35a in a third condition visually differentiated from the first and second conditions, to indicate ancestor-keys. The key background conditions can be differentiated by the colour of the key or of a portion of the key, or by appropriate texturing and or rendering of the key or of a portion of the key. In the enclosed embodiment three different colours have been used to identify a selected key, a son key and an ancestor key respectively.

Moreover, during the keys display step, the key shape 35b can also be changed depending upon the circumstances: ancestor-keys are displayed in a first shape and son-keys in a second shape visually differentiated from the first shape. Moreover the selected key can be configured according to the first shape, if the selected key has selectable son-keys, or according to said second shape, if the selected key has no selectable son-keys. The method provides for presence of sibling navigation keys: sibling keys are keys at the same level of the selected key in the hierarchical organization of the user interface. In the embodiment of the invention of the attached figures sibling keys are displayed only if the selected key has no son-keys. Said sibling keys are displayed in said second shape and with the same background colour of the son-keys. As shown in the embodiments of the attached figures and as mentioned when describing the user interface product, each key is tab-shaped: in such a case, the first shape is a first parallelogram and the second shape a second parallelogram. This second parallelogram has two parallel sides, both parallel to two corresponding parallel sides of the first parallelogram, and two sides oriented transversally relative to corresponding two parallel sides of the first parallelogram. Basically the parallelogram shaped tabs can have two shapes, which are different for the inclination of two of their four sides.

Finally a key identifier 35c, for instance an Icon, a wording, or an alphanumeric representation, can be displayed on the key surface for describing the function of or the information contained in the working display associated to each respective key. A further feature for identifying if one or more of said son-keys or sibling keys present on their turn respective son-keys. In the hierarchical organization son keys of son keys (or of sibling keys) are hidden when displaying the selected key in the navigation region. However, the control system is programmed to display an indicium, herein defined as indicium 35d, on each son-key or sibling key having second level son-keys so that a user can have a perception not only of the number and type keys selectable after having selected a certain key but also an information as to whether these keys have respective son-keys below (see figure 4).

## Claims

1. Medical apparatus comprising:
at least a user interface (30) for enabling setting of a plurality of parameters pertinent to operation of said apparatus or pertinent to a process to be performed by said apparatus, the user interface (30) including at least a screen (31),
a control system (18) for controlling operation of said medical apparatus and
responsive to actions by a user on said user interface (30),
said control system (18) being programmed for executing the following steps:
- defining on said screen (31) an operating region (32), where a number of working displays (36) can be displayed, and a navigation region (33), where a number of navigation keys (35) can be displayed.
- organizing said navigation keys (35) according to a multi-level hierarchical structure where son-keys (139) of a specific navigation key are defined as keys hierarchically one level below said specific key, and ancestor-keys (40, 41) of a specific key are defined as keys hierarchically one or more levels above the specific key;
- associating to each of said number of navigation keys (35) a corresponding working display:
- verifying selection of a navigation key;
- displaying in said navigation region (33) at least the following keys:
the key selected by the user,
if present, one or more son-keys (139) of the selected key (38), and
if present, one or more ancestor-keys (40, 41);
- displaying, in the operating region (32), at least the working display associated to the selected key (38),
wherein the control system (18) is further programmed to associate to each of said keys a number of properties including a key shape, and wherein during the keys display step the control system (18) is programmed to:
- display ancestor-keys (40, 41) of the selected key (38) according to a first shape and son-keys (139) of the selected key (38) according to a second shape visually differentiated from the first shape,
- configure the selected key (38) according to said first shape, if the selected key (38) has selectable son-keys (139), or according to said second shape, if the selected key (38) has no selectable son-keys (139), wherein each of said keys is tab-shaped, the first shape being a first parallelogram and the second shape being a second parallelogram having two parallel sides oriented transversally relative to corresponding two parallel sides of the first parallelogram.

2. Apparatus according to claim 1, wherein the multi-level hierarchical structure also provides for sibling keys (39) of a specific navigation key, which are defined as keys hierarchically at the same level of said specific key.

3. Apparatus according to claim 2, wherein during the keys display step the control system (18) is programmed to hide any sibling keys (39) of the selected key if the selected key has at least one son-key (139), and to display any sibling keys (39) of the selected key if the selected key has no son-keys.

4. Apparatus according to claim 1, wherein the control system (18) is programmed to allow selection of a son-key (139) of a specific key only after selection of the same specific key.

5. Apparatus according to claim 1, wherein during the keys display step the control system (18) is programmed to display the selected key (38) in a manner visually differentiated compared to the son-keys (139) and to the ancestors-keys (40, 41).

6. Apparatus according to claim 5, wherein during the keys display step the control system (18) is programmed to display the son-keys (139) in a manner visually differentiated compared to the ancestor-keys (40, 41).

7. Apparatus according to claim 2, wherein during the keys display step the control system (18) is programmed to display the selected key (38) in a manner visually differentiated compared to the sibling-keys (39) and to the ancestors-keys.

8. Apparatus according to claim 7, wherein during the keys display step the control system (18) is programmed to display the sibling-keys (39) in a manner visually differentiated compared to the ancestor-keys (40, 41).

9. Apparatus according to claim 4, wherein the control system (18) is programmed to:
keep track of the group of ancestor keys required to be selected to allow display of the selected key (38), and
display said group of ancestor-keys (40, 41) during the keys display step.

10. Apparatus according to claim 9, wherein during the keys display step the control system (18) is programmed to display said group of ancestor-keys (40, 41) in a position adjacent to the selected key (38), the closer the level of the ancestor key to the level of the selected key (38), the closer said ancestor key being displayed to the selected key (38).

11. Apparatus according to claim 9, wherein during the keys display step the control system (18) is programmed to display said group of ancestor-keys (40, 41) and said son-keys (139) In opposite and adjacent positions relative to the selected key (38).

12. Apparatus according to claim 1, wherein the control system (18) Is programmed to associate to each of said keys the number of properties including a key background (35a), and wherein during the keys display step the control system (18) is programmed to display the key background (35a) in a first condition, to indicate a selected key (38), and to display the key background (35a) in at least a second condition visually differentiated from the first condition, to indicate an unselected key (38).

13. Apparatus according to claim 1, wherein the control system (18) is programmed to associate to each of said keys the number of properties including a key background (35a), and wherein during the keys display step the control system (18) is programmed to display the key background (35a) in a first condition, to indicate a selected key (38), to display the key background (35a) in at least a second condition visually differentiated from the first condition, to indicate son-keys (139), and to display the key background (35a) in a third condition visually differentiated from the first and second conditions, to indicate ancestor-keys (40, 41).

14. Apparatus according to claim 2 and 13, wherein the control system (18) is programmed to display the key background (35a) in a fourth condition visually differentiated from the first, second and third conditions, to indicate sibling-keys (39) of the selected key (38).

15. Apparatus according to claim 2 and 13, wherein during the keys display step the control system (18) is programmed to display the key background (35a) in said second condition visually differentiated from the first and third conditions, to also indicate sibling-keys (39) of the selected key (38).

16. Apparatus according to claim 2, wherein during the keys display step the control system (18) is programmed to display ancestor-keys (40, 41) of the selected key (38) according to said first shape, and if the selected key (38) has no son-keys (139) to display both the selected key (38) and any sibling keys (39) according to a second shape visually differentiated from the first shape.

17. Apparatus according to claim 1, wherein the control system (18) is programmed to associate to each of said keys the number of properties including a key Identifier (35c), which can be one in the group comprising an icon, a wording, or an alphanumeric representation, and wherein during the keys-display step the key identifier (35c) is displayed on the key surface describing the function of or the information contained in the working display associated to each respective key.

18. Apparatus according to claim 1, wherein the control system (18) is programmed for:
identifying if one or more of the son-keys (139) of the selected key (38) present on their turn respective son-keys (139) which are hidden when displaying the selected key (38) in the navigation region (33),
displaying an indicium on each son-key having respective son-keys (139).

19. Apparatus according to claim 2, wherein the control system (18) is programmed for:
identifying if one or more of the sibling-keys (39) of the selected key (38) present on their turn respective son-keys (139) which are hidden when displaying the selected key (38) in the navigation region (33),
displaying an indicium on each sibling-key having respective son-keys (139).

20. Apparatus according to claim 2, wherein the control system (18) is programmed for:
detecting selection of one son-key of a selected key (38),
transforming the selected son-key into a newly selected key (38),
transforming the previously selected key (38) into an ancestor-key, displaying, if any, son-keys (139) of the newly selected key (38),
displaying any ancestors of the newly selected key (38),
if the newly selected key (38) has no son-keys (139), then displaying any sibling keys (39) of said newly selected key (38).

21. Apparatus according to claim 1, wherein the control system (18) is programmed for:
detecting selection of one ancestor-key of a selected key (38),
transforming said ancestor-key into a newly selected key (38),
transforming the previously selected key (38) into a son-key,
displaying son-keys (139) of the newly selected key (38),
displaying, if any, ancestors of the newly selected key (38).

22. Apparatus according to claim 1, wherein said screen (31) is a touch screen (31) including said operating and said navigation regions (32, 33), said control system (18) allowing selection of a key by detecting a touching of the corresponding area delimited by a key border in the navigation region (33).

23. Apparatus according to claim 1, wherein at least one of said working displays (36) comprises an indicium, for identifying a parameter a user intends to modify; said control system (18) being programmed for:
allowing selection of the indicium for identifying the parameter a user intends to modify,
allowing setting of a new value for said parameter;
storing the new value in a memory.

24. Apparatus according to claim 23 wherein:
the screen (31) comprises a touch screen (31),
and wherein said step of allowing selection of the indicium comprises detecting a
touching of said indicium,
said step of allowing setting of a parameter value comprises the following steps:
in response to said touching, invoking a data-entry display (42) on the touch screen (31) said data entry display (42) having at least two set buttons (43) and an enter key;
receiving a setting of a parametric value corresponding to the treatment parameter by touching one or more buttons of the data-entry display (42);
detecting the touching of said enter key for signifying setting has been completed and entering the set value.

25. Apparatus according to claim 23, wherein:
the screen (31) comprises a touch screen (31),
the user interface (30) comprises at least an enter hard key off of said touch screen (31) and connected to the user interface (30),
and wherein:
said step of allowing selection of the indicium comprises detecting a touching of said indicium,
said step of allowing setting of a parameter value comprises the following steps:
in response to said touching, invoking a data-entry display (42) on a region of the touch screen (31) said data entry display (42) having at least two set buttons (43);
receiving a setting of a parametric value corresponding to the treatment parameter by touching one or more buttons of the data-entry display (42);
detecting the touching of said enter key for signifying setting has been completed.

26. Apparatus according to claim 23, wherein:
the screen (31) comprises a touch screen (31),
and wherein said step of allowing selection of the indicium comprises detecting a touching of said indicium,
said step of allowing setting of a parameter value comprises the following steps:
in response to said touching, activating on the touch screen (31) at least two set buttons (43) and an enter key;
receiving a setting of a parametric value corresponding to the treatment parameter by touching one or more buttons;
detecting the touching of said enter key for signifying setting has been completed,

27. Medical apparatus according to claim 23, comprising:
a dialysis liquid preparation module for preparing dialysis liquid,
at least a waste line for receiving spent dialysate,
a blood removal tubing,
a blood return tubing, and
a blood treatment unit having a first chamber connected to dialysis liquid preparation module and to the waste line, and a second chamber connected to the blood removal tubing and to the blood return tubing, said first and second chamber being separated by a semipermeable membrane.

28. Medical apparatus according to claim 23, wherein the parameters are one or more selected in the group comprising:
Temperature of the dialysis liquid,
Conductivity of the dialysis liquid,
Electrolytes concentration of the dialysis liquid,
Flow rate of the dialysis liquid,
Flow rate of the spent dialysate,
Flow rate of the blood in the in one of said blood removal tubing and blood return tubing,
Ultrafiltration rate through the semipermeable membrane,
Net weight loss rate,
Treatment time,
Weight loss.

## Patentansprüche

1. Medizinische Vorrichtung, umfassend:
mindestens eine Benutzeroberfläche (30) zum Ermöglichen der Einstellung einer Vielzahl von Parametern, die einen Betrieb der Vorrichtung betreffen oder ein von der Vorrichtung auszuführendes Verfahren betreffen, wobei die Benutzeroberfläche (30) mindestens einen Bildschirm (31) und
ein Steuerungssystem (18) zur Steuerung des Betriebs der medizinischen Vorrichtung, das auf Handlungen eines Benutzers hinsichtlich der Benutzeroberfläche (30) reagiert, beinhaltet,
wobei das Steuerungssystem (18) programmiert ist, die folgenden Schritte auszuführen:
- Definieren eines Betriebsbereiches (32) auf dem Bildschirm (31), in dem eine Anzahl von Arbeitsanzeigen (36) angezeigt werden kann, sowie eines Navigationsbereiches (33), in dem eine Anzahl von Navigationstasten (35) angezeigt werden kann,
- Organisieren der Navigationstasten (35) gemäß einer mehrstufigen Hierarchiestruktur, in der Sohntasten (139) einer spezifischen Navigationstaste als Tasten definiert sind, die hierarchisch eine Stufe unter der spezifischen Taste sind, und Vorfahrentasten (40, 41) einer spezifischen Taste als Tasten definiert sind, die hierarchisch eine oder mehrere Stufen über der spezifischen Taste sind;
- Zuordnen einer entsprechenden Arbeitsanzeige zu jeder der Anzahl von Navigationstasten (35);
- vernizieren der Auswant einer Navigationstate;
- Anzeigen mindestens der folgenden Tasten im Navigationsbereich (33):
die vom Benutzer ausgewählte Taste,
wenn vorhanden, eine oder mehrere Sohntasten (139) der ausgewählten Taste (38) und
wenn vorhanden, eine oder mehrere Vorfahrentasten (40, 41);
- Anzeigen mindestens der Arbeitsanzeige im Arbeitsbereich (32),die der ausgewählten Taste (38) zugeordnet ist,
wobei das Steuerungssystem (18) weiterprogrammiert ist, jeder der Tasten eine Anzahl von Eigenschaften einschließlich einer Tastenform zuzuordnen, und wobei während des Tastenanzeigeschrittes das Steuerungssystem (18) programmiert ist zum:
- Anzeigen der Vorfahrentasten (40, 41) der ausgewählten Taste (38) gemäß einer ersten Form und Sohntasten (139) der ausgewählten Taste (38) gemäß einer sich optisch von der ersten Form unterscheidenden zweiten Form,
- Konfigurieren der ausgewählten Taste (38) gemäß einer ersten Form, wenn die ausgewählte Taste (38) auswählbare Sohntasten (139) aufweist oder gemäß der zweiten Form, wenn die ausgewählte Taste (38) keine auswählbaren Sohntasten (139) aufweist, wobei jede der Tasten registerkartenförmig ist, wobei die erste Form ein erstes Parallelogramm ist und die zweite Form ein zweites Parallelogramm ist, aufweisend zwei

2. Vorrichtung nach Anspruch 1, wobei die mehrstufige Hierarchiestruktur außerdem Geschwistertasten (39) einer spezifischen Navigationstaste vorsieht, die als Tasten definiert sind, die hierarchisch in derselben Stufe der spezifischen Taste liegen.

3. Vorrichtung nach Anspruch 2, wobei während des Tastenanzeigeschrittes das Steuerungssystem (18) programmiert ist, eventuelle Geschwistertasten (39) der ausgewählten Taste zu verbergen, wenn die ausgewählte Taste mindestens eine Sohntaste (139) aufweist, und eventuelle Geschwistertasten (39) der ausgewählten Taste anzuzeigen, wenn die ausgewählte Taste keine Sohntasten aufweist.

4. Vorrichtung nach Anspruch 1, wobei das Steuerungssystem (18) programmiert ist, die Auswahl einer Sohntaste (139) einer spezifischen Taste erst nach der Auswahl dieser spezifischen Taste zu gestatten.

5. Vorrichtung nach Anspruch 1, wobei während des Tastenanzeigeschrittes das Steuerungssystem (18) programmiert ist, die ausgewählte Taste (38) auf eine sich von den Sohntasten (139) und den Vorfahrentasten (40, 41) optisch unterscheidende Art und Weise anzuzeigen.

6. Vorrichtung nach Anspruch 5, wobei während des Tastenanzeigeschrittes das Steuerungssystem (18) programmiert ist, die Sohntasten (139) auf eine sich von den Vorfahrentasten (40, 41) optisch unterscheidende Art und Weise anzuzeigen.

7. Vorrichtung nach Anspruch 2, wobei während des Tastenanzeigeschrittes das Steuerungssystem (18) programmiert ist, die ausgewählte Taste (38) auf eine sich von den Geschwistertasten (39) und den Vorfahrentasten optisch unterscheidende Art und Weise anzuzeigen.

8. Vorrichtung nach Anspruch 7, wobei während des Tastenanzeigeschrittes das Steuerungssystem (18) programmiert ist, die Geschwistertasten (39) auf eine sich von den VorfahreMasten (40, 41) optisch unterscheidende Art und Weise anzuzeigen.

9. Vorrichtung nach Anspruch 4, wobei das Steuerungssystem (18) programmiert ist zum:
Merken der Gruppe von Vorfahrentasten, die ausgewählt werden muss, um eine Anzeige der ausgewählten Taste (38) zu gestatten und
Anzeigen der Gruppe von Vorfahrentasten (40, 41) während des Tastenanzeigeschrittes.

10. Vorrichtung nach Anspruch 9, wobei während des Tastenanzeigeschrittes das Steuerungssystem (18) programmiert ist, die Gruppe von Vorfahrentasten (40, 41) in einer Position anzuzeigen, die neben der ausgewählten Taste (38) liegt, wobei die Vorfahrentaste umso näher zur ausgewählten Taste (38) angezeigt wird, je näher die Stufe der Vorfehrentaste der Stufe der ausgewählten Taste (36) Ist.

11. Vorrichtung nach Anspruch 9, wobei während des Tastenanzeigeschrittes das Steuerungssystem (18) programmiert ist, die Gruppe von Vorfahrentasten (40, 41) und die Sohntasten (139) relativ zur ausgewählten Taste (38) in gegenüberliegenden und benachbarten Positionen anzuzeigen.

12. Vorrichtung nach Anspruch 1, wobei das Steuerungssystem (18) programmiert ist, jeder der Tasten die Anzahl von Eigenschaften einschließlich eines Tastenhintergrundes (35a) zuzuordnen, und wobei während des Tastenanzeigeschrittes das Steuerungssystem (18) programmiert ist, den Tastenhintergrund (35a) in einem ersten Zustand anzuzeigen, um eine ausgewählte Taste (38) abzubilden, und den Tastenhintergrund (35a) in mindestens einem sich vom ersten Zustand optisch unterscheidenden zweiten Zustand anzuzeigen, um eine nicht ausgewählte Taste (38) abzubilden.

13. Vorrichtung nach Anspruch 1, wobei das Steuerungssystem (18) programmiert ist, jeder der Tasten die Anzahl von Eigenschaften einschließlich eines Tastenhintergrundes (35a) zuzuordnen, und wobei während des Tastenanzeigeschrittes das Steuerungssystem (18) programmiert ist, den Tastenhintergrund (35a) in einem ersten Zustand anzuzeigen, um eine ausgewählte Taste (38) abzubilden, den Tastenhintergrund (35a) in mindestens einem sich vom ersten Zustand optisch unterscheidenden zweiten Zustand anzuzeigen, um Sohntasten (139) abzubilden, und den Tastenhintergrund (35a) in einem sich optisch vom ersten und zweiten Zustand unterscheidenden dritten Zustand anzuzeigen, um Vorfahrentasten (40, 41) abzubilden.

14. Vorrichtung nach den Ansprüchen 2 und 13, wobei das Steuerungssystem (18) programmiert ist, den Tastenhintergrund (35a) in einem sich vom ersten, zweiten und dritten Zustand optisch unterscheidenden vierten Zustand anzuzeigen, um Geschwistertasten (39) der ausgewählten Taste (38) abzubilden.

15. Vorrichtung nach den Ansprüchen 2 und 13, wobei während des Tastenanzeigeschrittes das Steuerungssystem (18) programmiert ist, den Tastenhintergrund (35a) im sich vom ersten und dritten Zustand optisch unterscheidenden zweiten Zustand anzuzeigen, um außerdem die Geschwistertasten (39) der ausgewählten Taste (38) abzubilden.

16. Vorrichtung nach Anspruch 2, wobei während des Tastenanzeigeschrittes das Steuerungssystem (18) programmiert ist, Vorfahrentasten (40, 41) der ausgewählten Taste (38) gemäß der ersten Form anzuzeigen, und wenn die ausgewählte Taste (38) keine Sohntasten (139) aufweist, sowohl die ausgewählte Taste (38) als auch eventuelle Geschwistertasten (39) gemäß einer sich von der ersten Form optisch unterscheidenden zweiten Form.

17. Vorrichtung nach Anspruch 1, wobei das Steuerungssystem (18) programmiert ist, jeder der Tasten eine Anzahl von Eigenschaften einschließlich eines Tastenbezeichners (35c) zuzuordnen, der einer in der Gruppe umfassend ein Symbol, ein Wort oder eine alphanumerische Darstellung sein kann, und wobei während des Tastenanzeigeschrittes der Tastenbezeichner (35c) auf der Tastenoberfläche angezeigt wird, beschreibend die Funktion der Arbeitsanzeige oder die darin enthaltene Information, die jeder entsprechenden Taste zugeordnet ist.

18. Vorrichtung nach Anspruch 1, wobei das Steuerungssystem (18) programmiert ist zum:
Ermitteln, ob eine oder mehrere der Sohntasten (139) der ausgewählten Taste (38) wiederum entsprechende Sohntasten (139) aufweisen, die verborgen sind, wenn die ausgewählte Taste (38) im Navigationsbereich (33) angezeigt wird,
Anzeigen einer Angabe auf jeder Sohntaste, die entsprechende Sohntasten (139) aufweist

19. Vorrichtung nach Anspruch 2, wobei das Steuerungssystem (18) programmiert ist zum:
Ermitteln, ob eine oder mehrere der Geschwistertasten (39) der ausgewählten Taste (38) wiederum entsprechende Sohntasten (139) aufweisen, die verborgen sind, wenn die ausgewählte Taste (38) im Navigationsbereich (33) angezeigt wird,
Anzeigen einer Angabe auf jeder Geschwistertaste, die entsprechende Sohntasten (139) aufweist.

20. Vorrichtung nach Anspruch 2, wobei das Steuerungssystem (18) programmiert ist zum:
Erfassen der Auswahl einer Sohntaste einer ausgewählten Taste (38),
Umwandeln der ausgewählten Sohntaste in eine neu ausgewählte Taste (38),
Umwandeln der zuvor ausgewählten Taste (38) in eine Vorfahrentaste, Anzeigen, wenn vorhanden, von Sohntasten (139) der neu ausgewählten Taste (38),
Anzeigen eventueller Vorfahren der neu ausgewählten Taste (38),
wenn die neu ausgewählte Taste (38) keine Sohntasten (139) aufweist, dann Anzeigen eventueller Geschwistertasten (39) der neu ausgewählten Taste (38).

21. Vorrichtung nach Anspruch 1, wobei das Steuerungssystem (18) programmiert ist zum :
Erfassen der Auswahl einer Vorfahrentaste einer ausgewählten Taste (38),
Umwandeln der ausgewählten Vorfahrentaste in eine neu ausgewählte Taste (38),
Umwandeln der zuvor ausgewählten Taste (38) in eine Sohntaste,
Anzeigen der Sohntasten (139) der neu ausgewählten Taste (38).
Anzeigen, wenn vorhanden, von Vorfahren der neu ausgewählten Taste (38).

22. Vorrichtung nach Anspruch 1, wobei der Bildschirm (31) ein Berührungsbildschirm (31) ist, einschließlich des Betriebs- und des Navigationsbereichs (32, 33), wobei das Steuerungssystem (18) die Auswahl einer Taste durch Erfassen einer Berührung des entsprechenden Feldes gestattet, das von einem Tastenrand im Navigationsbereich (33) begrenzt wird.

23. Vorrichtung nach Anspruch 1, wobei mindestens eine der Arbeitsanzeigen (36) eine Angabe zum Ermitteln eines Parameters umfasst, den ein Benutzer ändern möchte, wobei das Steuerungssystem (18) programmiert ist zum:
Gestatten einer Auswahl der Angabe zum Ermitteln des Parameters, den ein Benutzer ändern möchte,
Gestatten des Einstellens eines neuen Wertes für den Parameter;
Speichern des neuen Wertes in einem Speicher.

24. Vorrichtung nach Anspruch 23 wobei der Bildschirm (31) einen Berührungsbildschirm (31) umfasst,
und wobei der Schritt des Gestattens der Auswahl der Angabe das Erfassen einer Berührung der Angabe umfasst,
wobei der Schritt des Gestattens des Einstellens eines Parameterwertes die folgenden Schritte umfasst:
Aufrufen einer Dateneingabeanzeige (42) auf dem Berührungsbildschirm (31) in Reaktion auf die Berührung, wobei die Dateneingabeanzeige (42) mindestens zwei Einstellschaltflächen (43) und eine Eingabetaste aufweist;
Empfangen einer Einstellung eines dem Behandlungsparameter entsprechenden parametrischen Wertes durch Berühren einer oder mehrerer Schaltflächen der Dateneingabeanzeige (42);
Erfassen der Berührung der Eingabetaste, um zu kennzeichnen, dass die Einstellung abgeschlossen wurde, und den Sollwerte einzugeben.

25. Vorrichtung nach Anspruch 23, wobei:
der Bildschirm (31) einen Berührungsbildschirm (31) umfasst,
die Benutzeroberfläche (30) mindestens einen Eingabe-Hardkey außerhalb des Berührungsbildschirms (31) umfasst, der mit der Benutzeroberfläche (30) verbunden ist,
und wobei:
der Schritt des Gestattens der Auswahl der Angabe das Erfassen einer Berührung der Angabe umfasst,
wobei der Schritt des Gestattens des Einstellens eines Parameterwertes die folgenden Schritte umfasst:
Aufrufen einer Dateneingabeanzeige (42) auf einem Bereich des Berührungsbildschirms (31) in Reaktion auf die Berührung, wobei die Dateneingabeanzeige (42) mindestens zwei Einstellschaltflächen (43) aufweist;
Empfangen einer Einstellung eines dem Behandlungsparameter entsprechenden parametrischen Wertes durch Berühren einer oder mehrerer Schaltflächen der Dateneingabeanzeige (42);
Erfassen der Berührung der Eingabetaste, um zu kennzeichnen, dass die Einstellung abgeschlossen wurde.

26. Vorrichtung nach Anspruch 23, wobei:
der Bildschirm (31) einen Berührungsbildschirm (31) umfasst und wobei der Schritt des Gestattens der Auswahl der Angabe das Erfassen einer Berührung der Angabe umfasst,
wobei der Schritt des Gestattens des Einstellens eines Parameterwertes die folgenden Schritte umfasst:
Aktivieren von mindestens zwei Einstellschaltflächen (43) und einer Eingabetaste auf dem Berührungsbildschirm (31) in Reaktion auf die Berührung;
Empfangen einer Einstellung eines dem Behandlungsparameter enteprechenden parametriechen Wortoo duroh Berühren einer oder mehrerer Schaltflächen;
Erfassen der Berührung der Eingabetaste, um zu kennzeichnen, dass die Einstellung abgeschlossen wurde.

27. Medizinische Vorrichtung nach Anspruch 23, umfassend:
ein Dialyseflüssigkeitsvorbereitungsmodul zur Vorbereitung einer Dialyseflüssigkeit,
mindestens eine Entsorgungsleitung zum Empfang des verwendeten Dialysats,
eine Schlauchleitung zum Entfernen von Blut,
eine Schlauchleitung zum Rückführen von Blut,
eine Blutbehandlungseinheit, aufweisend eine erste Kammer, die mit einem Dialyseflüssigkeitsvorbereitungsmodul und mit der Entsorgungsleitung verbunden ist, und eine zweite Kammer, die mit der Schlauchleitung zum Entfernen von Blut und mit der Schlauchleitung zum Rückführen von Blut verbunden ist, wobei die erste und zweite Kammer durch eine semipermeable Membran getrennt sind.

28. Medizinische Vorrichtung nach Anspruch 23, wobei die Parameter einer oder mehrere sind, ausgewählt aus der Gruppe umfassend:
die Temperatur der Dialyseflüssigkeit,
die Leitfähigkeit der Dialyseflüssigkeit,
die Elektrolytkonzentration der Dialyseflüssigkeit,
die Durchflussrate der Dialyseflüssigkeit,
die Durchflussrate des verwendeten Dialysats,
die Durchflussrate des Blutes in einer der Schlauchleitungen zum Entfernen von Blut und Schlauchleitungen zum Rückführen von Blut,
die Ultrafiltrationsrate durch die semipermeable Membran,
die Nettogewichtverlustrate,
die Behandlungszeit,
den Gewichtsverlust.

## Revendications

1. Appareil médical comprenant:
au moins une interface utilisateur (30) pour permettre le réglage d'une pluralité de paramètres pertinents pour le fonctionnement dudit appareil ou
pertinents pour un processus devant être exécuté par ledit appareil,
l'interface utilisateur (30) comprenant au moins un écran (31),
un système de commande (18) pour commander le fonctionnement dudit appareil médical et étant sensible aux actions de la part d'un utilisateur sur ladite interface utilisateur (30),
ledit système de commande (18) étant programmé pour exécuter les étapes suivantes:
- définir sur ledit écran (31) une région de fonctionnement (32), où un nombre d'afficheurs de fonctionnement (36) peut s'afficher, et une région de navigation (33), où un nombre de touches de navigation (35) peut s'afficher,
- organiser lesdites touches de navigation (35) selon une structure hiérarchique à plusieurs niveaux où des touches-filles (139) d'une touche de navigation spécifique sont définies comme des touches se situant du point de vue hiérarchique à un niveau en dessous de ladite touche spécifique, et des touches-ancêtres (40, 41) d'une touche spécifique sont définies comme des touches se situant du point de vue hiérarchique à un ou plusieurs niveaux au-dessus de la touche spécifique;
- associer à chacune dudit nombre de touches de navigation (35) un afficheur de fonctionnement correspondant;
- vérifier la sélection de la touche de navigation;
- afficher dans ladite région de navigation (33) au moins une des touches suivantes:
la touche sélectionnée par l'utilisateur,
le cas échéant, une ou plusieurs touches-filles (139) de la touche sélectionnée (38), et
le cas échéant, une ou plusieurs touches-ancêtres (40, 41):
- afficher, dans la région de fonctionnement (32), au moins l'afficheur de fonctionnement associé à la touche sélectionnée (38),
dans lequel le système de commande (18) est également programmé pour associer à chacune desdites touches un nombre de propriétés comprenant une forme de touche, et dans lequel, durant l'étape d'afficher des touches, le système de commande (18) est programmé pour:
- afficher des touches-ancêtres (40, 41) de la touche sélectionnée (38) selon une première forme et des touches-filles (139) de la touche sélectionnée (38) selon une deuxième forme se différenciant visuellement de la première forme,
- configurer la touche sélectionnée (38) selon une première forme, si la touche sélectionnée (38) présente des touches-filles sélectionnables (139), ou selon ladite deuxième forme, si la touche sélectionnée (38) présente des touches-filles non sélectionnables (139), dans lequel chacune des dites touches a la forme d'une languette, la première forme étant un premier parallélogramme et la deuxième forme étant un deuxième parallélogramme ayant deux côtés parallèles orientés transversalement par rapport aux deux côtés parallèles correspondants du premier parallélogramme.

2. Appareil selon la revendication 1, dans lequel la structure hiérarchique à plusieurs niveaux prévoit également des touches-soeurs (39) d'une touche de navigation spécifique, qui sont définies comme des touches hiérarchiquement au même niveau que ladite touche spécifique.

3. Appareil selon la revendication 2, dans lequel durant l'étape d'afficher les touches le système de commande (18) est programmé pour cacher toutes les touches-soeurs (39) de la touche sélectionnée si la touche sélectionnée présente au moins une touche-fille (139), et pour afficher toutes les touches-soeurs (39) de la touche sélectionnée si la touche sélectionnée ne présente pas de touches-filles.

4. Appareil selon la revendication 1, dans lequel le système de commande (18) est programmé pour permettre la sélection d'une touche-fille (139) d'une touche spécifique uniquement après la sélection de la même touche spécifique.

5. Appareil selon la revendication 1, dans lequel durant l'étape d'afficher les touches le système de commande (18) est programmé pour afficher la touche sélectionnée (38) d'une manière se différenciant visuellement des touches-filles (139) et des touches-ancêtres (40, 41).

6. Appareil selon la revendication 5, dans lequel durant l'étape d'afficher les touches le système de commande (18) est programmé pour afficher les touches-filles (139) d'une manière se différenciant visuellement des touches-ancêtres (40, 41).

7. Appareil selon la revendication 2, dans lequel durant l'étape d'afficher les touches le système de commande (18) est programmé pour afficher la touche sélectionnée (38) d'une manière se différenciant visuellement des touches-soeurs (39) et des touches-ancêtres.

8. Appareil selon la revendication 7, dans lequel durant l'étape d'afficher les touches le système de commande (18) est programmé pour afficher les touches-soeurs (39) d'une manière se différenciant visuellement des touches-ancêtres (40, 41).

9. Appareil selon la revendication 4, dans lequel le système de commande (18) est programmé pour:
garder une trace du groupe de touches-ancêtres nécessaires pour être sélectionnées afin de permettre l'affichage de la touche sélectionnée (38),
et afficher ledit groupe de touches-ancêtres (40, 41) durant l'étape d'affichage des touches.

10. Appareil selon la revendication 9, dans lequel durant l'étape d'afficher les touches le système de commande (18) est programmé pour afficher ledit groupe de touches-ancêtres (40, 41) dans une position adjacente à la touche sélectionnée (38), plus le niveau de la touche-ancêtre est proche du niveau de la touche sélectionnée (38), plus la touche ancêtre affichée est proche de la touche sélectionnée (38).

11. Appareil selon la revendication 9, dans lequel durant l'étape d'afficher les touches le système de commande (18) est programmé pour afficher ledit groupe de touches-ancêtres (40, 41) et lesdites touches-filles (139) dans des positions opposées et adjacentes à la touche sélectionnée (38).

12. Appareil selon la revendication 1, dans lequel le système de commande (18) est programmé pour associer à chacune desdites touches le nombre de propriétés comprenant un arrière-plan de la touche (35a), et dans lequel durant l'étape d'affichage des touches le système de commande (10) première condition, pour indiquer une touche sélectionnée (35a) dans une première condition, pour indiquer une touche sélectionnée (38), et pour afficher l'arrière-plan de la touche (35a) dans au moins une deuxième condition se différenciant visuellement de la première condition, pour indiquer une touche non sélectionnée (38).

13. Appareil selon la revendication 1, dans lequel le système de commande (18) est programmé pour associer à chacune desdites touches le nombre de propriétés comprenant un arrière-plan de la touche (35a), et dans lequel durant l'étape d'affichage des touches le système de commande (18) est programmé pour afficher l'arrière-plan de la touche (35a) dans une première condition, pour indiquer une touche sélectionnée (38), pour afficher l'arrière-plan de la touche (35a) dans au moins une deuxième condition se différenciant visuellement de la première condition, pour indiquer des touches-filles (139), et pour afficher l'arrière-plan de la touche (35a) dans une troisième condition se différenciant visuellement de la première et de la deuxième condition, pour indiquer des touches-ancêtres (40,41).

14. Appareil selon les revendications 2 et 13, dans lequel le système de commande (18) est programmé pour afficher l'arrière-plan de la touche (35a) dans une quatrième condition se différenciant visuellement de la première, de la deuxième et de la troisième condition, pour indiquer des touches-soeurs (39) de la touche sélectionnée (38).

15. Appareil selon les revendications 2 et 13, dans lequel durant l'étape d'affichage des touches le système de commande (18) est programmé pour afficher l'arrière-plan de la touche (35a) dans ladite deuxième condition se différenciant visuellement de la première et de la troisième condition, pour indiquer également des touches-soeurs (39) de la touche sélectionnée (38).

16. Appareil selon la revendication 2, dans lequel durant l'étape d'afficher les touches le système de commande (18) est programmé pour afficher des touches-ancêtres (40, 41) de la touche sélectionnée (38) selon ladite première forme, et si la touche sélectionnée (38) ne présente pas de touches-filles (139) pour afficher la touche sélectionnée (38) et toutes les touches-soeurs (39) selon une deuxième forme se différenciant visuellement de la première forme.

17. Appareil selon la revendication 1, dans lequel le système de commande (18) est programmé pour associer à chacune desdites touches le nombre de propriétés comprenant un identificateur de touche (35c), qui peut être un identificateur du groupe comprenant une icône, une formulation ou une représentation alphanumérique, et dans lequel durant l'étape d'affichage des touches l'identificateur de touche (35c) est affiché sur la surface de la touche décrivant la fonction ou l'information présente sur l'afficheur de fonctionnement associé à chaque touche respective.

18. Appareil selon la revendication 1, dans lequel le système de commande (18) est programmé pour:
identifier si une ou plusieurs touches-filles (139) de la touche sélectionnée (38) présentent à leur tour des touches-filles respectives (139) qui sont cachées à l'affichage de la touche sélectionnée (38) dans la région de navigation (33),
afficher une indication sur chaque touche-fille présentant des touches-filles respectives (139).

19. Appareil selon la revendication 2, dans lequel le système de commande (18) est programmé pour:
identifier si une ou plusieurs touches-soeurs (39) de la touche sélectionnée (38) présentent à leur tour des touches-filles respectives (139) qui sont cachées à l'affichage de la touche sélectionnée (38) dans la région de navigation (33),
afficher une indication sur chaque touche-soeur présentant des touches-filles respectives (139).

20. Appareil selon la revendication 2, dans lequel le système de commande (18) est programmé pour:
détecter la sélection d'une touche-fille d'une touche (38) sélectionnée,
transformer la touche-fille en une touche (38) récemment sélectionnée,
transformer la touche (38) sélectionnée précédemment en une touche-ancêtre,
afficher, le cas échéant, des touches-filles (139) de la touche (38) récemment sélectionnée,
afficher tous les ancêtres de la touche (38) récemment sélectionnée,
si la touche (38) récemment sélectionnée ne présente pas de touches-filles (139), afficher toutes les touches-soeurs (39) de ladite touche (38) récemment sélectionnée.

21. Appareil selon la revendication 1, dans lequel le système de commande (18) est programmé pour:
détecter la sélection d'une touche-ancêtre d'une touche sélectionnée (38),
transformer ladite touche-ancêtre en une touche (38) récemment sélectionnée,
transformer la touche (38) sélectionnée précédemment en une touche-fille,
afficher des touches-filles (139) de la touche (38) récemment sélectionnée,
afficher, le cas échéant, des ancêtres de la touche (38) récemment sélectionnée.

22. Appareil selon la revendication 1, dans lequel ledit écran (31) est un écran tactile (31) comprenant la dite région de fonctionnement et ladite région de navigation (32, 33), ledit système de commande (18) permettant la sélection d'une touche en détectant un effleurement de la zone correspondante délimitée par un bord de touche dans la région de navigation (33).

23. Appareil selon la revendication 1, dans lequel au moins un desdits afficheurs de fonctionnement (36) comprend une indication pour identifier un paramètre qu'un utilisateur a l'intention de modifier; ledit système de commande (18) étant programmé pour:
permettre la sélection d'une indication pour identifier le paramètre qu'un utilisateur a l'intention de modifier,
permettre le réglage d'une nouvelle valeur pour ledit paramètre ;
stocker la nouvelle valeur dans une mémoire.

24. Appareil selon la revendication 23, dans lequel:
l'écran (31) comprend un écran tactile (31),
et dans lequel ladite étape de permettre la sélection de l'indication comprend la détection d'un effleurement de ladite indication,
ladite étape de permettre le réglage d'une valeur de paramètre comprend les étapes suivantes:
en réponse audit effleurement, invoquant un afficheur d'entrée des données (42) sur l'écran tactile (31) ledit afficheur d'entrée des données (42) présentant au moins deux boutons de réglage (43) et une touche entrée;
recevoir un réglage d'une valeur paramétrique correspondant au paramètre de traitement en effleurant un ou plusieurs boutons de
l'afficheur d'entrée des données (42);
détecter l'effleurement de ladite touche entrée pour indiquer que le réglage a été achevé et entrer la valeur de réglage.

25. Appareil selon la revendication 23, dans lequel:
l'écran (31) comprend un écran tactile (31),
l'interface utilisateur (30) comprend au moins une touche entrée non programmable à l'extérieur dudit écran tactile (31) et reliée à l'interface utilisateur (30),
et dans lequel:
ladite étape de permettre la sélection de l'indication comprend la détection d'un effleurement de ladite indication,
ladite étape de permettre le réglage d'une valeur de paramètre comprend
les étapes suivantes:
en réponse audit effleurement, invoquant un afficheur d'entrée des données (42) sur une région de l'écran tactile (31), ledit afficheur d'entrée des données (42) présentant au moins deux boutons de réglage (43);
recevoir un réglage d'une valeur paramétrique correspondant au paramètre de traitement en effleurant un ou plusieurs boutons de l'afficheur d'entrée des données (42);
détecter l'effleurement de ladite touche entrée pour indiquer que le réglage a été achevé.

26. Appareil selon la revendication 23, dans lequel:
l'écran (31) comprend un écran tactile (31),
et dans lequel ladite étape de permettre la sélection de l'indication comprend la détection d'un effleurement de ladite indication,
ladite étape de permettre le réglage d'une valeur de paramètre comprend les étapes suivantes:
en réponse audit effleurement, activant sur l'écran tactile (31) au moins deux boutons de réglage (43) et une touche entrée;
recevoir un réglage d'une valeur paramétrique correspondant au paramètre de traitement en effleurant un ou plusieurs boutons;
détecter l'effleurement de ladite touche entrée pour indiquer que le réglage a été achevé.

27. Appareil médical selon la revendication 23, comprenant:
un module de préparation du liquide de dialyse pour préparer le liquide de dialyse,
au moins une conduite d'évacuation pour recevoir le liquide de dialyse usé,
un tube d'élimination du sang,
un tube de retour du sang, et
une unité de traitement du sang présentant une première chambre reliée au module de préparation du liquide de dialyse et à la conduite d'évacuation, et une deuxième chambre reliée aux tubes d'élimination du sang et de retour du sang, lesdites première et deuxième chambres étant séparées par une membrane semi-perméables.

28. Appareil médical selon la revendication 23. dans lequel un ou plusieurs paramètres sont sélectionnés dans le groupe comprenant:
température du liquide de dialyse,
conductivité du liquide de dialyse,
concentration en électrolytes du liquide de dialyse,
débit du liquide de dialyse,
débit du liquide de dialyse usé,
débit du sang dans un desdits tubes d'élimination du sang et de retour du sang,
taux d'ultrafiltration à travers la membrane semi-perméable,
taux de perte de poids net,
temps de traitement,
perte de poids.
